# EUROPEAN PATENT SPECIFICATION

(11) **EP 2 514 410 B1**
(45) Date of publication and mention of the grant of the patent: **04.05.2016**
(21) Application number: 10837779.7
(22) Date of filing: 04.11.2010
(51) Int. Cl.: A61K 39/09, A61K 9/127, A61K 47/30, A61K 47/48

(54) **LIPOSOME COMPOSITION, PREPARATION METHOD THEREOF, AND USAGE OF THE COMPOSITION AS PNEUMONIA VACCINE**
LIPOSOMZUSAMMENSETZUNG, HERSTELLUNGSVERFAHREN DAFÜR UND VERWENDUNG DER ZUSAMMENSETZUNG ALS IMPFSTOFF GEGEN LUNGENENTZÜNDUNG
COMPOSITION LIPOSOMALE, SON PROCÉDÉ DE PRÉPARATION ET UTILISATION DE CETTE COMPOSITION COMME VACCIN CONTRE LA PNEUMONIE

(30) Priority: 16.12.2009 KR 20090125698; 28.10.2010 KR 20100106035
(43) Date of publication of application: 24.10.2012
(73) Proprietor: Jeonnam Bioindustry Foundation, Jeollanam-Do 520-330 (KR); Jeongpil Corp., Jeollanam-Do 519-951 (KR)
(72) Inventor: PARK, Sin Jeong, Gwangju-city 503-230 (KR); HAN, Sang In, Incheon-city 406-743 (KR); SEO, Kook Heon, Hwasun-gun Jeollanam-Do 519-800 (KR); KIM, Yang Jin, Hwasun-gun Jeollanam-Do 519-767 (KR); KIM, Jin Nam, Gwangju-city 502-752 (KR); CHOI, Myeong Jun, Seongnam-si Gyeonggi-Do 463-734 (KR)
(74) Representative: Betten & Resch
(86) International application number: PCT/KR2010/007762
(87) International publication number: WO 2011/074782

(56) References cited:
- WO-A1-2007/039583
- WO-A1-2007/039584
- KR-A- 20080 081 063
- US-A1- 2004 043 954
- US-A1- 2008 171 053
- ABRAHAM E: "INTRANASAL IMMUNIZATION WITH BACTERIAL POLYSACCHARIDE CONTAINING LIPOSOMES ENHANCES ANTIGEN-SPECIFIC PULMONARY SECRETORY ANTIBODY RESPONSE", VACCINE, vol. 10, no. 7, 1992, pages 461-468, XP23711055, ISSN: 0264-410X
- CHANG, J. S ET AL.: 'Development of Thl-mediated CD8+ effector T cells by vaccination with epitope peptides encapsulated in pH-sensitive liposomes.' VACCINE. vol. 19, no. 27, 14 June 2001, pages 3608 - 3614, XP004241777
- ALVING, C. R ET AL.: 'Lipid A and liposomes containing lipid A as antigens and adjuvants.' VACCINE. vol. 26, no. 24, 26 December 2007, pages 3036 - 3045, XP022703341

## Description

### Technical Field

The present invention relates to a vaccine composition for the prophylaxis of pneumonia, comprising a pneumococcal capsular polysaccharide. More particularly, the present invention relates to a vaccine composition for the prophylaxis of pneumonia, comprising pneumococcal capsular polysaccharide antigen-encapsulated, immunopotentiating liposomes free of carrier proteins.

### Background Art

*Streptococcus pneumoniae* (pneumococcus) is part of the normal upper respiratory tract flora, with causing no symptoms, but as with many natural flora, it can become pathogenic under the right conditions, causing pneumonia and many types of pneumococcal infections including meningitis, otitis media, and sinusitis. *Streptococcus pneumoniae* is currently known as the leading cause of invasive bacterial disease, such as pneumonia, in children. More than 90 different serotypes of *S. pneumoniae* are known. Most prevalent in pneumonia in children are *S. pneumoniae* serotypes 4, 6B, 9V, 14, 18C, 19F and 23F: these seven serotypes are estimated to be responsible for about 70% of invasive infections in children. There are currently pneumococcal conjugate vaccines available on the global market: one is Prevenar-7, manufactured by Wyeth. It is a heptavalent vaccine containing the pneumococcal capsular polysaccharides of the seven serotypes of *S. pneumoniae*, linked to CRM₁₉₇, a nontoxic recombinant variant of diphtheria toxin. Synflorix, produced by GlaxoSmithKline, is a decavalent pneumococcal conjugate vaccine which contains antigen from ten pneumococcal serotypes: serotypes 1, 5 and 7F, plus the seven that are contained in Prevenar-7. It is recommended for children from six weeks to two years of age against invasive pneumococcal diseases and acute otitis media. Recently, Wyeth introduced Prevenar 13 which contains pneumococcal capsular polysaccharides of six additional serotypes (1, 3, 5, 6A, 19A and 7F) plus the seven of Prevenar-7.

The pneumococcal vaccines developed by Wyeth and GSK are characterized by pneumococcal capsular polysaccharides conjugated with the diphtheria toxoid CRM₁₉₇. Because the pneumococcal capsular polysaccharides themselves are not sufficient to stimulate T cells, the diphtheria toxoid is recruited. Particularly for infants and children, who have insufficiently developed T cells, proteins that can stimulate T cells are necessary, in addition to the pneumococcal capsular polysaccharides. Recent reports have it that conjugate vaccines in which pneumococcal capsular polysaccharides are conjugated with such carrier proteins are likely to induce asthma (reactive airway disease) in children. Therefore, a novel pneumonia vaccine with reduced risk for such side effects is required particularly for children.

As a strategic approach for the development of capsular polysaccharide vaccines, a liposomal drug delivery system has attracted intense attention. A liposome is an artificially prepared vesicle composed of phospholipids, found in biological membranes and is widely used as a drug delivery system or to deliver an antigen. Much research has been studied on liposome vaccines containing peptide and protein antigens (Brgles, M et al. Vaccine 2009, 27, 5435-5442; Jain, V et al. J Drug Target 2008, 16, 706-715; Alving & Rao Vaccine 2008, 26, 3036-3045; Arigita, C et al. Vaccine 2005, 23, 5091-5098; Chang, JS et al. Vaccine 2001, 19, 3608-3614; Chang, JS et al. Vaccine 1999, 17. 1540-1548; Van Cott, JL et al. Vaccine 1996, 14, 392-398), but only a little research done on liposome vaccines containing capsular polysaccharides. In recent years, liposomes with polysaccharide antigens have been improved in immunogenicity by encapsulating proteins (alpha-toxin, haemagglutinin) as well thereinto (Burgeot, C et al, Vaccine 2001, 19, 2092-2099; Pietrobon, P.J.F. et al, Immunomethods 1994, 4, 236-243). Lipoxen developed a multivalent liposomal composition two or more polysaccharide antigens and a protein carrier (ovalbumin, tetanus toxoid, Diphtheria toxoid, diphtheria CRM197 (a genetic mutant of diphtheria toxin)), wherein the polysaccharide antigens are co-entrapped within liposomes with the protein carrier (WO 2007/039584 A1), without a linkage between the polysaccharide and the protein carrier.

A carrier protein that is encapsulated along with an antigen has an economical disadvantage in that it increases the production cost and time. In addition, it may act as an allergen.

### Disclosure

### Technical Problem

Culminating in the present invention, intensive and thorough research into a vaccine composition for the prophylaxis of pneumonia, conducted by the present inventors, led to the finding that a liposome with a specific phospholipid and cholesterol composition enhances the immunogenicity of pneumococcal capsular polysaccharides sufficiently not to recruit a carrier protein.

It is therefore an object of the present invention to provide a liposome composition for the prevention of pneumonia, with pneumococcal capsular polysaccharides encapsulated thereinto, without a carrier protein.

It is another object of the present invention to provide a method for preparing the liposome composition.

It is a further object of the present invention to provide the use of the liposome composition in the construction of a vaccine composition for the prevention of pneumonia.

### Technical Solution

In order to accomplish the above objects, the present invention provides a liposome composition, comprising a pneumococcal capsular polysaccharide antigen-encapsulated antigen liposomes, said liposomes containing no carrier proteins and being made of:
a phospholipid selected from the group consisting of phosphatidylcholine, phosphatidylethanolamine and a combination thereof; and
a cholesterol selected from the group consisting of cholesterol, cholesterol hemisuccinate, a derivative thereof, and a combination thereof.

The liposome composition of the present invention may be prepared by a method for preparing the liposome composition of any one of claims 1 to 9, comprising:
mixing a solution of empty liposomes with a pneumococcal capsular polysaccharide antigen to form a mixture of liposome and antigen; and
repeating a cycle of freezing the mixture in liquid nitrogen and thawing at room temperature.

In addition, the present invention addresses a vaccine composition for prophylaxis of pneumonia, comprising pneumococcal capsular polysaccharide antigen-encapsulated liposomes, said liposomes containing no carrier proteins and being made of:
a phospholipid selected from the group consisting of phosphatidylcholine, phosphatidylethanolamine and a combination thereof; and
a cholesterol selected from the group consisting of cholesterol, cholesterol hemisuccinate, a derivative thereof, and a combination thereof.

Below, a detailed description will be given of the present invention.

Unless otherwise stated, all the technical terms used herein have the same meanings as are understood to those having ordinary skill in the art to which the present invention pertains. Although preferable methods and materials are described herein, those similar or equivalent to the methods and materials fall within the scope of the present invention.

After intensive and thorough studies on a vaccine for the prophylaxis of pneumonia, the present inventors found that when pneumococcal capsular polysaccharides are entrapped within a liposome having a specific composition, their immunogenicity is potentiated sufficiently to elicit immunity against pneumonia in a subject without the aid of a carrier protein, so that the liposome with pneumococcal capsular polysaccharides entrapped thereinto, a carrier protein being neither linked to the pneumococcal capsular polysaccharides nor co-entrapped into the liposome, can be useful as a vaccine for the prophylaxis of pneumonia. In addition, it was unexpectedly and surprisingly found that pneumococcal capsular polysaccharides are more greatly potentiated in immunogenicity when they are used in combination with a liposome having a specific phospholipid and cholesterol composition than when they are linked to a carrier protein or co-entrapped with a carrier protein within a typical liposome. The immunopotentiating liposome comprises a phospholipid selected from the group consisting of phosphatidylcholine, phosphatidylethanolamine and a combination thereof; and a cholesterol selected from the group consisting of cholesterol, Cholesterol hemisuccinate, derivatives thereof, and a combination thereof.

In accordance with an aspect thereof, the present invention provides a liposome composition comprising pneumococcal capsular polysaccharide antigen-encapsulated liposomes, said liposomes containing no carrier proteins and being made of:
a phospholipid selected from the group consisting of phosphatidylcholine, phosphatidylethanolamine and a combination thereof; and
a cholesterol selected from the group consisting of cholesterol, cholesterol hemisuccinate, a derivative thereof, and a combination thereof.

In accordance with one embodiment of the present invention, the liposomes may further comprise an adjuvant selected from the group consisting of monophosphoryl lipid A (MPL), muramyl dipeptide (MDP), poloxamer (PX), and a combination thereof.

The liposome may be charged negatively, positively or neutrally on the surface thereof. When it is anionic, the liposome may comprise phosphatidylethanolamine, cholesterol hemisuccinate, and monophosphoryl lipid A (MPL) at a weight ratio of 6 ~ 8: 2 ~ 4 : 0.02 ~ 0.2. Most preferably, the anionic liposome may comprise phosphatidylethanolamine, cholesterol hemisuccinate, and monophosphoryl lipid A (MPL) at a weight ratio of approximately 7 : 3 : 0.035.

When the liposome is charged positively, it may comprise phosphatidylcholine, cholesterol, stearyl amine and monophosphoryl lipid A (MPL) at a weight ratio of 5 ~ 7: 2 ~ 4 : 0.5 ~ 2 : 0.02 ~ 0.2. In the cationic liposome, phosphatidylcholine, most preferably, cholesterol, stearyl amine and monophosphoryl lipid A (MPL) may be used at a weight ratio of 6 : 3 : 1 : 0.03.

When it is electrically neutral, the liposome may comprise phosphatidylcholine, cholesterol and monophosphoryl lipid A (MPL) at a weight ratio of 5 ~ 7: 2 ~ 4 : 0.02 - 0.2. Most preferably, the neutral liposome comprises phosphatidylcholine, cholesterol and monophosphoryl lipid A (MPL) at a weight ratio of 6 : 3 : 0.03.

Abraham (Vaccine, 10(7):461-468, 1992) describes liposomes comprising phosplatidylcholine, cholesterol and phosphatidylserine which contain encapsulated pneumococcal PS. In the liposome, the pneumococcal capsular polysaccharide is entrapped at a weight ratio of 1:50~250 of phospholipid. The liposome ranges in average diameter from 50 to 2000 nm, preferably from 50 to 1000 nm, more preferably from 60 to 700 nm, far more preferably from 80 to 500 nm, and even far more preferably from 100 to 300 nm. The average diameter may be measured by PCS (photon correlation spectroscopy), as disclosed in WO 99/65465.

The liposome composition may be in the form of a suspension in which liposomes are dispersed in a continuous phase of aqueous medium. Alternatively, the liposome composition may be in the form of powder after, for example, spray drying or freeze drying, and if necessary, may be diluted in water or an aqueous liquid to form an aqueous dispersion.

In accordance with another aspect thereof, the present invention provides a method for preparing a liposome composition, comprising:
mixing a solution of empty liposomes with a pneumococcal capsular polysaccharide antigen to form a mixture of liposomes and antigen; and
repeating a freezing-thawing cycle in which the mixture is frozen in liquid nitrogen and then thawed at room temperature.

The empty liposomes may be small or medium in size with a structure of unilamellar vesicles (SUV) or multilamella vesicles (MLV). The MLV or SUV may be prepared from such a specific composition as is described above, using a typical method known in the art. For example, lipids are dissolved in an organic solvent (e.g., chloroform) which is then removed using nitrogen gas to form a thin lipid film on the wall of the glass vessel. Phosphate buffered saline is added to the glass vessel and vigorously stirred to form such vesicles. The MLV thus obtained is ultrasonicated using a tip-type sonicator to form unilamellar vesicles. The resulting empty liposomes are homogenized in size by passing them through a membrane filter with a predetermined pore size, for example, about 0.2 ~ 2 µm. The homogenized empty liposomes are mixed with a pneumococcal capsular polysaccharide antigen and the mixture is subjected to many cycles of freezing in liquid nitrogen and thawing at room temperature to afford liposomes within which the pneumococcal capsular polysaccharide antigen is encapsulated.

The method for preparing liposomes for use in the vaccine composition of the present invention is not limited to the freezing-thawing method. An extrusion method or a dehydration-rehydration method may be employed.

According to the extrusion method, liposomes may be prepared as follows: MLV, after being prepared in the same manner as described for the freezing-thawing method, is put into an extruder equipped with membrane filters of various sizes and extruded by nitrogen gas at a high pressure to pass through the membranes to afford suitable sizes of liposomes. In this method, the MLV is extruded through a 3.0 µm membrane, and then through 1.0, 0.4, and 0.2 µm membranes in the order. In addition, the liposomes, after coming out of a 0.1 µm membrane, may be subjected to the freezing-thawing method.

According to the dehydration-rehydration method, liposomes may be prepared as follows:The MLV which is prepared in the same manner as is described for the freezing-thawing method is changed into unilamellar vesicles by sonication. These unilamellar vesicles are mixed with a capsular polysaccharide and trehalose, followed by lyophilization. The resulting lyophilized liposomes may be stored. For use, they may be rehydrated in distilled water.

After pneumococcal capsular polysaccharide antigens are encapsulated into the liposomes, the drugs remaining unencapsulated or too large liposomes may be removed or the liposomes may be reduced in mean size according to a method known in the art.

Thus, the pneumococcal capsular polysaccharide antigen-encapsulated liposomes may range in mean size from 50 to 2,000 nm, preferably from 50 to 1,000 nm, more preferably from 60 to ~ 700 nm, far more preferably from 80 to 500 nm, and even far more preferably from 100 to 300 nm. Hence, the liposome composition according to the present invention preferably excludes antigen-encapsulated liposomes larger than 2,000 nm or smaller than 20 nm.

In accordance with a further aspect thereof, the present invention provides the use of the liposome composition of the present invention in preparing a vaccine for the prophylaxis of pneumonia which is designed to elicit an immune response to a pneumococcal capsular polysaccharide in a mammal.

Thus, one aspect of the present invention addresses a vaccine composition for the prophylaxis of pneumonia, comprising a pneumococcal capsular polysaccharide-encapsulated liposome, said liposome containing no carrier proteins therein and being made of:
a phospholipid selected from the group consisting of phosphatidylcholine, phosphatidylethanolamine and a combination thereof; and
a cholesterol selected from the group consisting of cholesterol, cholesterol hemisuccinate, a derivative thereof, and a combination thereof.

In one embodiment, the vaccine composition for the prophylaxis of pneumonia may further comprise an adjuvant selected from the group consisting of monophosphoryl lipid A (MPL), muramyl dipeptide (MDP), poloxamer(PX), and a combination thereof.

Also, the vaccine composition for the prophylaxis of pneumonia may further a pharmaceutically acceptable diluent or excipient. The vaccine composition for the prophylaxis of pneumonia may be formulated into dosage forms suitable for subcutaneous injection, intraperitoneal injection, intramuscular injection, intranasal administration, inhalation or oral administration. The pharmaceutically acceptable diluent or excipient is selected suitably depending on the administration route or dosage form and is known in the art.

Preferably, the immune response comprises the production of IgG against the pneumococcal capsular polysaccharide antigen plus the production of IgM and IgA, thereby vaccinating the subject against pneumococcal infections.

When compared with a pneumococcal capsular polysaccharide antigen conjugate with a carrier protein, a liposome encapsulating the conjugate, and a liposome with a carrier protein and a pneumococcal capsular polysaccharide co-entrapped thereinto, the vaccine composition for the prophylaxis of pneumonia, comprising a pneumococcal capsular polysaccharide antigen-encapsulated liposome in accordance with the present invention was found to assure greater antibody production than did those containing a carrier protein (see Experimental Example 2, infra). This effect is too remarkable for those skilled in the art to expect in consideration of the role of the carrier protein as an adjuvant. Therefore, the vaccine composition according to the present invention can allow the production of antibodies in an amount large enough to use it as a vaccine preventive of pneumonia.

### Advantageous Effects

When a pneumococcal capsular polysaccharide is encapsulated thereinto, as described hitherto, the immunopotentiating liposome with a specific lipid composition is useful as an active ingredient for a vaccine composition for the prophylaxis of pneumonia, demonstrating that a vaccine with a high titer can be prepared from only pneumococcal capsular polysaccharides with the aid of an immunopotentiating liposome. Accordingly, because it does not necessitate a carrier protein, the vaccine of the present invention is more economically beneficial in terms of production cost and time, compared to conventional pneumonia vaccines using a carrier protein. In addition, the vaccine composition of the present invention, free of a carrier protein, can be safely inoculated to those who exhibit allergy to proteins.

### Description of Drawings

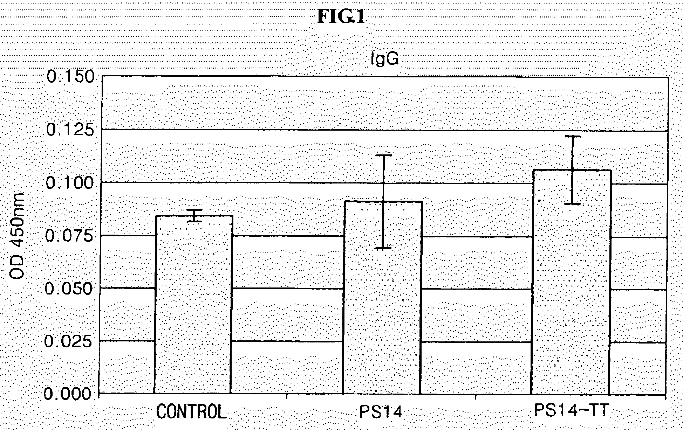

FIG. 1 is a graph showing antibody titers measured on day 42 after the first vaccination (two weeks after the third vaccination) in mice which has been immunized three times every two weeks with the capsular polysaccharide derived from pneumococcal serotype 14 or with the capsular polysaccharide-tetanus toxoid conjugate.

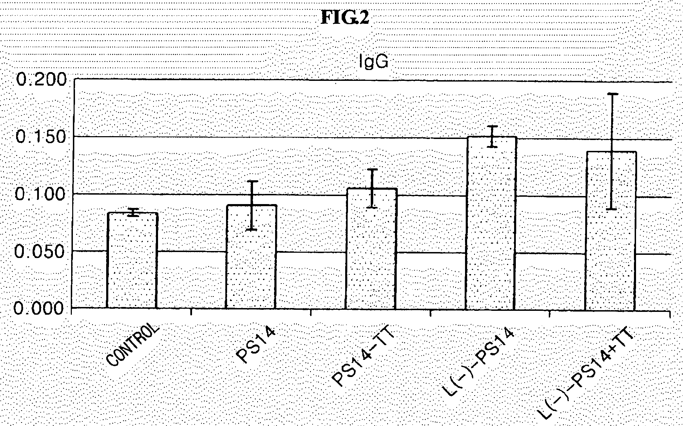

FIG. 2 is a graph showing serum antibody titers measured in the mice administered every two weeks three times with serotype 14 capsular polysaccharide (PS14), a capsular polysaccharide-tetanus toxoid conjugate (PS14-TT), liposomes within which a mixture of capsular polysaccharide and tetanus toxoid was entrapped (L(-)-PS14+TT), and the anionic liposomes (L(-)-PS14) of the present invention, on day 42 after the first immunization.

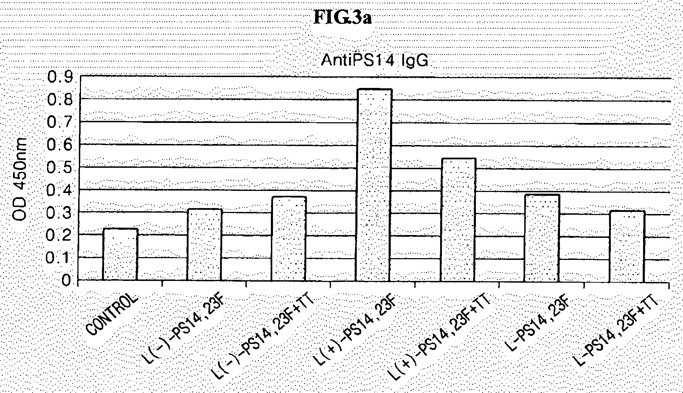

FIG. 3a is a graph showing IgG titers against PS14 measured in mice immunized three times every two weeks with the capsular polysaccharide-encapsulated anionic liposome antigen (L(-)-PS14,23F) (Example 4), the cationic liposome antigen with a mixture of capsular polysaccharide and tetanus toxoid entrapped therein (L(+)-PS14,23F+TT) (Comparative

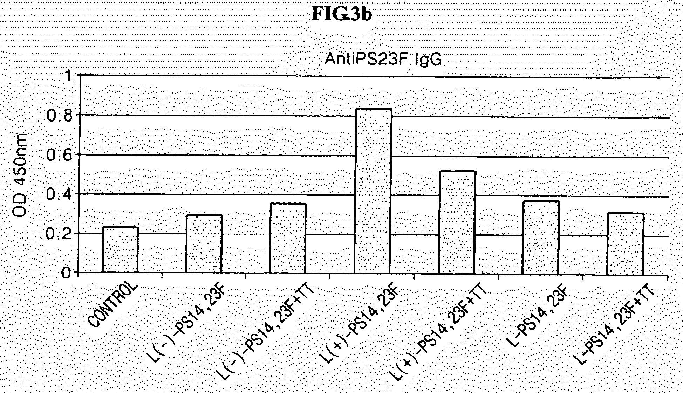

FIG. 3b is a graph showing IgG titers against PS23F measured in mice immunized three times every two weeks with the capsular polysaccharide-encapsulated anionic liposome antigen (L(-)-PS14,23F) (Example 4), the cationic liposome antigen with a mixture of capsular polysaccharide and tetanus toxoid entrapped therein (L(+)-PS14,23F+TT) (Comparative Example 3), the capsular polysaccharide-entrapped cationic liposome antigen (L(+)-PS14,23F) (Example 5), the cationic liposome antigen with a mixture of capsular polysaccharide and tetanus toxoid entrapped therein (L(+)-PS14,23F+TT) (Comparative Example 4), the capsular polysaccharide-encapsulated neutral liposome antigen (L-PS14,23F) (Example 6), the neutral liposome antigen with a mixture of capsular polysaccharide and tetanus toxoid entrapped therein (L-PS14,23F+TT) (Comparative Example 5), on day 42 after the first immunization.

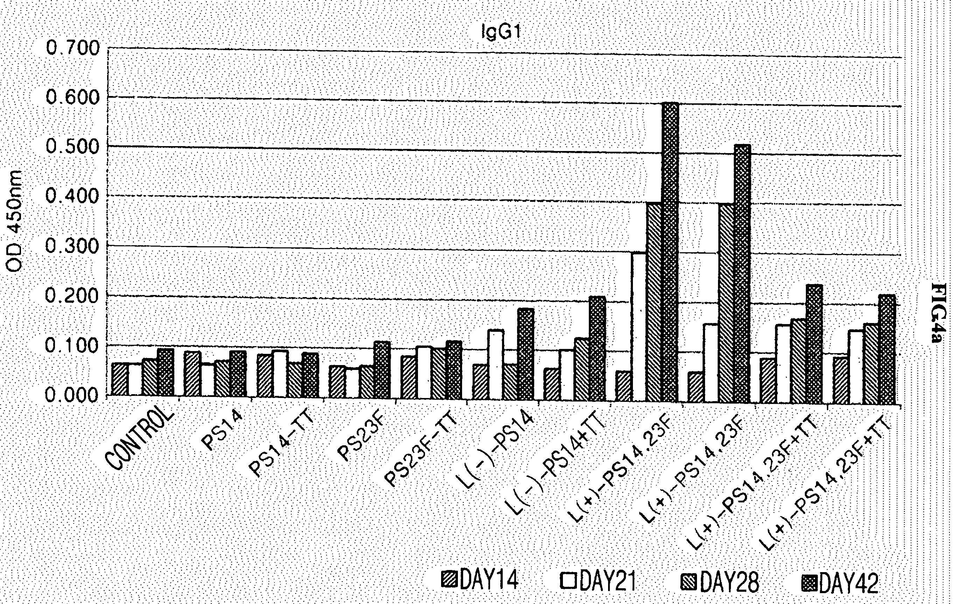

FIG. 4a is a graph showing IgG1 titers measured in mice immunized three times every two weeks with the liposomes prepared above, on days 14, 21, 28 and 42 after the first immunization.

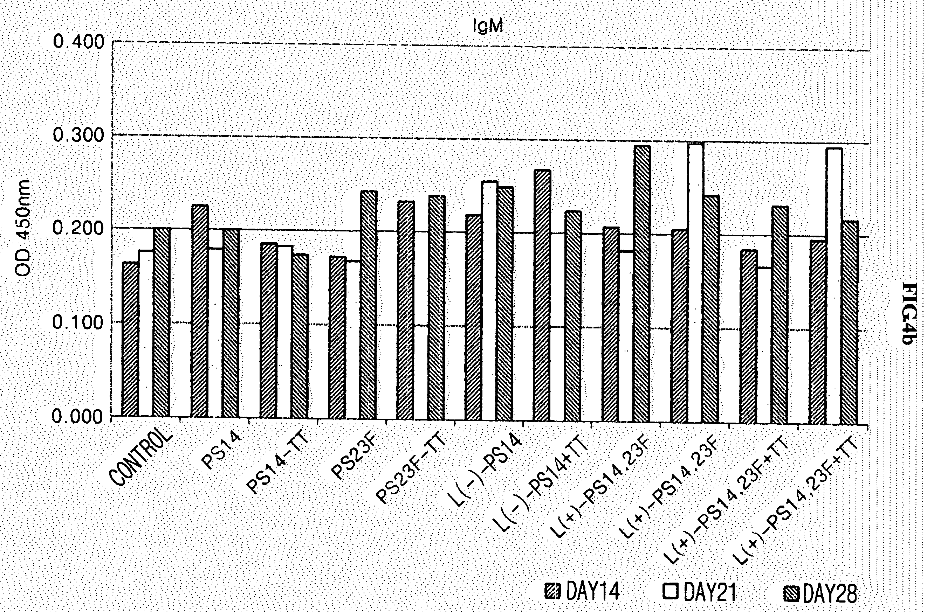

FIG. 4b is a graph showing IgM titers measured in mice immunized three times every two weeks with the liposomes prepared above, on days 14, 21 and 28 after the first immunization.

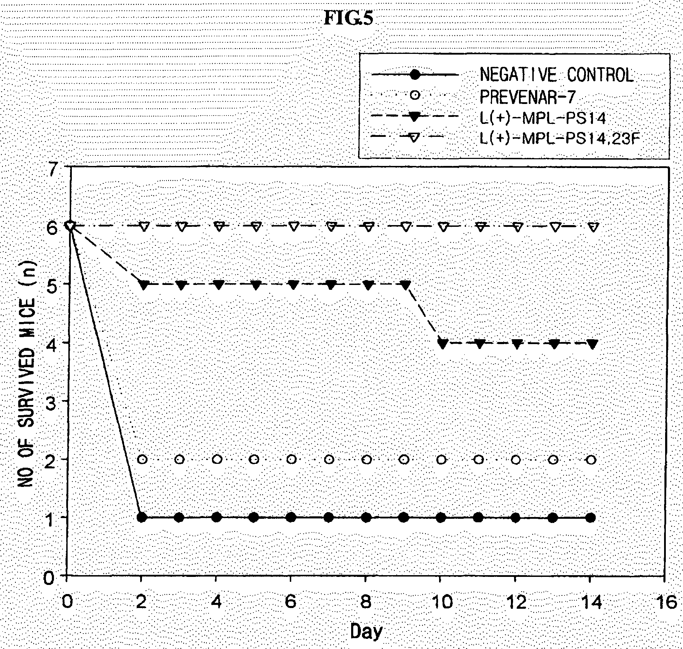

FIG. 5 is a graph showing the numbers of mice which survived over time after injection of *Streptococcus pneumoniae* serotype 14 three weeks post-immunization with the liposomes of Examples and Prevenar-7 three times every two weeks

### Mode for Invention

A better understanding of the present invention may be obtained through the following examples.

### EXAMPLES 1 TO 6: Preparation of Immunopotentiating Liposomes (1)

### 1) Preparation of capsular polysaccharide antigen

After *Streptococcus pneumoniae* serotype 14 (*Streptococcus pneumoniae* (Klein) Chester ATCC 6314) was cultured in a 2-liter fermenter, a capsular polysaccharide was separated from the bacteria using an alcohol precipitation method. The separated capsular polysaccharide was quantitatively analyzed using the phenol-sulfuric acid method while protein quantification was conducted using the Lowry method. After quantitative analysis of proteins and capsular polysaccharides, the sample was used as a PS14 capsular polysaccharide antigen.

The same procedure was repeated for *Streptococcus pneumoniae* serotype 23F *(Streptococcus pneumoniae* (Klein) Chester ATCC) to give a PS23F capsular antigen.

### 2) Preparation of liposome composition

PS14-encapsulated liposomes that were different in surface charge (negatively charged liposomes (Example 1), positively charged liposomes (Example 2), neutrally charged liposomes (Example 3)) were prepared.

Anionic liposomes were prepared from phosphatidyl ethanolalime (PE) (70 mg)/cholesterol hemisuccinate (CHOH) (30 mg), cationic liposomes from phosphatidylcholine (PC) (60 mg)/cholesterol (CHOL) (30 mg)/stearyl amine (SA) (1 mg), and neutral liposomes from PC (60 mg) / CHOL (30 mg). As an adjuvant, MPL (monophosphoryl lipid A) was used in a small amount in preparing each liposome. The components and their weight ratios used in the preparation of each liposome are summarized in Table 1, below.

**TABLE 1**

| Component | Weight Ratio (w/w) | | |
|---|---|---|---|
| | Anionic Liposome | Cationic Liposome | Neutral Liposome |
| PC (Phosphatidylcholine) | 0 | 6 | 6 |
| PE | 7 | 0 | 0 |
| (Phosphatidylethanolamine) | | | |
| CHOL (Chloesterol) | 0 | 3 | 3 |
| CHOH (Chloesterol hemisuccinate) | 3 | 0 | 0 |
| SA (Stearylamine) | 0 | 1 | 0 |
| MPL (Monophosphoryl lipid A) | 0.035 | 0.03 | 0.03 |

The components for each liposome were dissolved in the indicated amounts in chloroform which was then removed using nitrogen gas. When the solvent was removed by blowing nitrogen gas over the chloroform solution, the phospholipids were thinly spread on the wall of the glass vessel. After the formation of a thin film of phospholipids on the wall of the glass vessel, 10 mL of phosphate buffered saline was added and vigorously stirred. In this regard, the phospholipid film was completely detached from the wall and dispersed uniformly in the phosphate buffered saline to form multilamellar vesicles (MLV). The MLV was sonicated using a tip-type sonicator (VC505, SONICS) to afford small unilamellar vesicles (SUV) that were then allowed to pass through a 0.2 µm membrane filter. This filtered liposome solution was mixed at a weight ratio of 200:1 with the capsular polysaccharide antigen (PS14) and slightly stirred at room temperature. This mixed solution was subjected to three rounds of freezing at about -178°C in liquid nitrogen and thawing at room temperature. As a result, PS14-encapsulated anionic liposomes (Example 1, L(-)-MPL-PS14), cationic liposomes (Example 2, L(+)-MPL-PS14), and neutral liposomes (Example 3, L-MPL-PS14) were yielded.

Separately, the liposome solution obtained after passage through the membrane filter was mixed at a weight ratio of 200:1:1 with the capsular polysaccharide antigens PS14 and PS23F. The mixed solution was subjected to three rounds of freezing at about -178°C in liquid nitrogen and thawing at room temperature. As a result, liposomes which had both PS14 and PS23F entrapped therein and were anionic (Example 4, L(-)-MPL-PS14,23F), cationic (Example 5, L(+)-MPL-PS14,23F), and neutral (Example 6, L-MPL-PS14,23F) were yielded.

### EXAMPLES 7 AND 8: Preparation of Immunopotentiating Liposomes (2)

Cationic liposomes, within which both PS14 and PS23F were entrapped, were prepared in the same manner as in Example 5 (L(+)-MPL-PS14, 23F), with the exception that MDP (muramyl dipeptide) (Example 7, L(+)-MDP-PS14, 23F) or PX (Example 8, L(+)-PX-PS14, 23F) were used instead of MPL(monophosphozyl lipid A).

### EXAMPLE 9: Preparation of Immunopotentiating Liposomes (3)

Cationic liposomes within which PS14 and PS23F were co-entrapped (Example 9, L(+)-O-PS14, 23F) were prepared in the same manner as in Example 5, with the exception that none of the adjuvants such as MPL (monophosphoryl lipid A) were used.

COMPARATIVE EXAMPLES 1 AND 2: Preparation of Anionic Liposomes Having a Conjugate or Mixture of Antigen PS14 and Tetanus Toxoid Entrapped therein

Anionic liposomes within which both PS14 and tetanus toxoid were entrapped as a conjugate (Comparative Example 1, L(-)-PS14-TT) or in mixture (Comparative Example 2, L(-)-PS14+TT) were prepared in a manner similar to that of Example 1. The antigen was mixed at a weight ratio of 2:1 with tetanus toxoid. A conjugate of the capsular polysaccharide and tetanus toxoid (PS14-TT) was prepared using the Random CNBr activation Method.

COMPARATIVE EXAMPLE 3: Preparation of Anionic Liposomes Having a Mixture of PS14, PS23F and Tetanus Toxoid Entrapped therein

Anionic liposomes within which the antigens PS14 and PS23F and tetanus toxoid were entrapped (L(-)-PS14, PS23F+TT) were prepared in a manner similar to that of Example 4. The antigens were mixed at a weight ratio of 1:2 with tetanus toxoid.

COMPARATIVE EXAMPLE 4: Preparation of Cationic Liposomes Having a Mixture of PS14, PS23F and Tetanus Toxoid Entrapped therein

Cationic liposomes within which the antigens PS14 and PS23F and tetanus toxoid were entrapped (L(+)-PS14, PS23F+TT) were prepared in a manner similar to that of Example 5.

COMPARATIVE EXAMPLE 5: Preparation of Neutral Liposomes Having a Mixture of PS14, PS23F and Tetanus Toxoid Entrapped therein

Neutral liposomes within which the antigens PS14 and PS23F, and tetanus toxoid were co-entrapped (L-PS14, PS23F+TT) were prepared in a manner similar to that of Example 6.

### EXPERIMENTAL EXAMPLES

### Experimental Methods

### 1) Administration to positive control

For primary vaccination, FCA (Freund's Complete Adjuvant) (Sigma) was employed, followed by secondary and tertiary immunization with the aid of FIA (Freund's Incomplete Adjuvant) (Sigma). The antigen and the adjuvant were mixed at a volume ratio of 1:1 before use.

### 2) Immunization schedule

BALB/C female mice which were aged 12 weeks were used for immunity. The antigen (capsular polysaccharide PS14) was subcutaneously injected at a dose of 5 µg three times every two weeks into each mouse. Two weeks after the final injection, mice were sacrificed from which whole blood was then taken.

### 3) Measurement of antibody titer

On days 14 and 28 after the first vaccination, whole blood samples were taken from the tail veins of the mice and centrifuged at 4°C and 12,000 rpm for 5 min to give sera.

On day 42 after the first vaccination (2 weeks after the final injection), the mice were anesthetized with ether and underwent a laparotomy to sample blood from the heart.

The antibody titers (IgG, IgG1, IgG2a, IgA and IgM) of the sera were measured by ELISA. In this regard, each serum was 1:100 diluted in PBS-T (Phosphate Buffered Saline with 0.05% Tween 20) before use in ELISA. Absorbance at 450 nm (OD) was measured to compare antibody titers between groups.

### EXPERIMENTAL EXAMPLE 1: Antibody Production According to Kind of Antigen

The pneumococcal capsular polysaccharide (PS14) obtained from *Streptococcus pneumoniae* serotype 14 and the conjugate of the capsular polysaccharide and tetanus toxoid (PS14-TT) were evaluated for antibody productivity. Mice were immunized three times with the antigens. For this, they were used in mixture with the FCA adjuvant (1:1 v/v) for primary vaccination and in mixture with the FIA adjuvant (1:1, v/v) for secondary and tertiary vaccination. On 42 days after the first immunization, antibody titers were measured. The capsular polysaccharide-tetanus toxoid conjugate (PS14-TT) was prepared using the Random CNBr activation Method. The results are shown in FIG. 1.

FIG. 1 is a graph showing IgG titers measured on day 42 after the first vaccination (two weeks after the third vaccination) in mice which has been immunized three times every two weeks with the capsular polysaccharide derived from pneumococcal serotype 14 or with the capsular polysaccharide-tetanus toxoid conjugate.

As can be seen in FIG. 1, there is no significant difference in antibody titer according to the kind of antigen and no significant enhancement in antibody production was found between before and after the vaccination.

### EXPERIMENTAL EXAMPLE 2: Effect of Liposome in Antibody Production

The serotype 14 capsular polysaccharide (PS14) prepared in Example 1 and the capsular polysaccharide-tetanus toxoid conjugate (PS14-TT) prepared in Experimental Example 1 were injected into mice according to the administration method for positive control described above. The liposomes prepared in Comparative Example 1, having the capsular polysaccharide-tetanus toxoid conjugate trapped therein (L(-)-PS14-TT), the liposomes prepared in Comparative Example 2, having the mixture of capsular polysaccharide and tetanus toxoid trapped therein (L(-)-PS14+TT), and the anionic liposomes (L(-)-PS14) prepared in Example 1 were injected three times at regular intervals of two weeks into rats. On day 42 after the first immunization (two weeks after the final third immunization), serum antibody titers were measured. The results are shown in Table 2, below.

**TABLE 2**

| Serum Dilution Fold | Ab Production (OD) | | | | |
|---|---|---|---|---|---|
| | PS14 | PS14-TT | Ex. 1 L(-)-PS14 | C. Ex. 1 L(-)-PS14-TT | C. Ex. 2 L(-)-PS14+TT |
| 2 | 0.157 | 0.141 | 0.203 | 0.205 | 0.211 |
| 4 | 0.112 | 0.096 | 0.159 | 0.142 | 0.149 |
| 8 | 0.089 | 0.080 | 0.165 | 0.117 | 0.132 |
| 16 | 0.078 | 0.075 | 0.127 | 0.099 | 0.111 |
| 32 | 0.080 | 0.079 | 0.122 | 0.119 | 0.117 |
| 64 | 0.078 | 0.078 | 0.095 | 0.090 | 0.098 |
| 128 | 0.062 | 0.071 | 0.093 | 0.081 | 0.083 |
| 256 | 0.062 | 0.068 | 0.079 | 0.066 | 0.072 |

Separately, the serotype 14 capsular polysaccharide (PS14) prepared in Example 1, the capsular polysaccharide-tetanus conjugate (PS14-TT) prepared in Experimental Example 1 were injected into mice according to the administration method for positive control described above. The liposomal antigen, prepared in Comparative Example 2, having a mixture of capsular polysaccharide and tetanus toxoid entrapped therein (L(-)-PS14+TT), and the anionic liposome prepared in Example 1 (L(-)-PS14) were immunized every two weeks three times into mice. On day 42 after the first immunization (two weeks after the final third immunization), serum antibody titers were measured. The results are shown in FIG. 2.

FIG. 2 is a graph showing serum antibody titers measured in the mice administered every two weeks three times with serotype 14 capsular polysaccharide (PS14), a capsular polysaccharide-tetanus toxoid conjugate (PS14-TT), liposomes within which a mixture of capsular polysaccharide and tetanus toxoid was entrapped (L(-)-PS14+TT), and the anionic liposomes (L(-)-PS14) of the present invention, on day 42 after the first immunization.

As is understood from the data of Table 2 and FIG. 2, the vaccines comprising liposomes within which the antigens were entrapped (L(-)-PS14, L(-)-PS14-TT) L(-)-PS14+TT) assures significantly high antibody titers, compared to the vaccines comprising the antigens in mixture with FCA/FIA (PS14, PS14-TT conjugate). Different antibody titers were detected depending on whether the antigen PS14 was used alone or in combination with tetanus toxoid. Particularly comparing the liposomes having antigens encapsulated therein, higher antibody titers were produced by the liposomes within which the capsular polysaccharide antigen was entrapped alone (L(-)-PS14) than as a conjugate with tetanus toxoid (L-PS14-TT) or in mixture with tetanus toxoid (L(-)-PS14+TT). This effect was unexpected by those skilled in the art in light of the common sense that a carrier protein increases immunogenicity. From these results, it is understood that the liposome composition of the present invention can be used as a pneumonia vaccine that does not need a carrier protein, whether conjugated or co-entrapped.

### EXPERIMENTAL EXAMPLE 3: Antibody Productivity According to Surface Charge of Liposome

An examination was made of the antibody productivity according to the phospholipid composition, particularly, surface charge of liposome. In this context, anionic, cationic and neutral liposomes within which the antigens PS14 and PS23F were entrapped as prepared in Examples 4 to 6, and the liposomes within which the antigens PS14 and PS23F were entrapped together with tetanus toxoid as prepared in Comparative Examples 3 to 5 were used.

The antigens were used at the same doses according to the same immunization schedule. Mice were immunized three times every two weeks. On day 42 after the first immunization, serum samples were taken and diluted 1:100 before the measurement of antibody titer. The results are shown in FIGS. 3a and 3b.

FIG. 3a is a graph showing IgG titers against PS14 measured in mice immunized three times every two weeks with the capsular polysaccharide-encapsulated anionic liposome antigen (L(-)-PS14,23F) (Example 4), the anionic liposome antigen with a mixture of capsular polysaccharide and tetanus toxoid entrapped therein (L(-)-PS14,23F+TT) (Comparative Example 3), the capsular polysaccharide-entrapped cationic liposome antigen (L(+)-PS14,23F) (Example 5), the cationic liposome antigen with a mixture of capsular polysaccharide and tetanus toxoid entrapped therein (L(+)-PS14,23F+TT) (Comparative Example 4), the capsular polysaccharide-encapsulated neutral liposome antigen (L-PS14,23F) (Example 6), the neutral liposome antigen with a mixture of capsular polysaccharide and tetanus toxoid entrapped therein (L-PS14,23F+TT) (Comparative Example 5), on day 42 after the first immunization.

FIG. 3b is a graph showing IgG titers against PS23F measured in mice immunized three times every two weeks with the capsular polysaccharide-encapsulated anionic liposome antigen (L(-)-PS14,23F) (Example 4), the anionic liposome antigen with a mixture of capsular polysaccharide and tetanus toxoid entrapped therein (L(-)-PS14,23F+TT) (Comparative Example 3), the capsular polysaccharide-entrapped cationic liposome antigen (L(+)-PS14,23F) (Example 5), the cationic liposome antigen with a mixture of capsular polysaccharide and tetanus toxoid entrapped therein (L(+)-PS14,23F+TT) (Comparative Example 4), the capsular polysaccharide-encapsulated neutral liposome antigen (L-PS14,23F) (Example 6), the neutral liposome antigen with a mixture of capsular polysaccharide and tetanus toxoid entrapped therein (L-PS14,23F+TT) (Comparative Example 5), on day 42 after the first immunization.

As can be understood in FIG. 3a, the anti-PS14 IgG was produced at higher levels when using cationic liposomes than anionic liposomes.

The same production pattern was observed for the antibody against the serotype 23F capsular polysaccharide, as shown in FIG. 3b.

Separately, production levels of antibody isotypes was measured according to the phospholipid composition, particularly, surface charge of liposome. For this, the cationic liposome with both PS14 and PS23F encapsulated thereinto (Example 5), the cationic liposome with a mixture of PS14, PS23 and tetanus toxoid encapsulated thereinto (Comparative Example 4), the PS14-encapsualted anionic liposome (Example 1), and the anionic liposome with a mixture of PS14 and tetanus toxoid encapsulated thereinto (Comparative Example 2, L(-)-PS14+TT) were injected into mice.

As a positive control, PS14, a PS14-TT conjugate, PS23F, or a PS23F-TT conjugate was injected in combination with FCA/FIA according to the administration method for positive control described above.

The antigens were used at the same doses according to the same immunization schedule. Mice were immunized three times every two weeks. On days 14, 21, 28 and 42 after the first immunization, serum samples were taken and diluted 1:100 before the measurement of the titers of antibody isotypes. The results are shown in FIGS. 4a and 4b.

FIG. 4a is a graph showing IgG1 titers measured in mice immunized three times every two weeks with the liposomes prepared above, on days 14, 21, 28 and 42 after the first immunization.

FIG. 4b is a graph showing IgM titers measured in mice immunized three times every two weeks with the liposomes prepared above, on days 14, 21 and 28 after the first immunization.

Taken together, the data obtained above indicate that the cationic liposomes increase the immunogenicity of capsular polysaccharides irrespective of the kinds of the capsular polysaccharides and guarantee higher antibody titers in the absence of a carrier protein. Therefore, the liposome composition within which capsular polysaccharides, but not a carrier protein, are encapsulated can be used effectively as a pneumonia vaccine.

### EXPERIMENTAL EXAMPLE 4: Antibody Productivity According to Kind of Adjuvant

An examination was made of the antibody productivity according to the liposome composition, particularly the kind of adjuvant. Cationic liposomes with different adjuvants, prepared in Examples 5 and 7 to 9, were injected in the same manner as in Experimental Example 3.

Titers of total IgG, IgG1 and IgG2 against PS14 were measured on day 42 after the first immunization and the results are shown in Table 3, below. Titers of total IgG, IgG1 and IgG2 against PS23F are summarized in Table 4, below.

**TABLE 3**

| Ab | Ab Production (OD 450nm) | | | | |
|---|---|---|---|---|---|
| | Control (FCA) | L(+)-MPL-PS14,23F | L(+)-MDP-PS14,23F | L(+)-PX-PS14,23F | L (+) -O-PS14,23F |
| Total IgG | 0.101 | 0.142 | 0.145 | 0.166 | 0.131 |
| IgG1 | 0.063 | 0.157 | 0.228 | 0.202 | 0.162 |
| IgG2a | 0.067 | 0.108 | 0.088 | 0.016 | 0.062 |

**TABLE 4**

| Ab | Ab Production (OD 450nm) | | | | |
|---|---|---|---|---|---|
| | Control (FCA) | L(+)-MPL-PS14,23F | L(+)-MDP-PS14,23F | L(+)-PX-PS14,23F | L(+)-O-PS14,23F |
| Total IgG | 0.098 | 0.142 | 0.145 | 0.160 | 0.125 |
| IgG1 | 0.065 | 0.169 | 0.234 | 0.205 | 0.175 |
| IgG2a | 0.066 | 0.113 | 0.085 | 0.057 | 0.055 |

As is understood from the data of Tables 3 and 4, the liposome composition of the present invention induced antibody production without a significant difference according to the kind of the adjuvants. Further, the liposome devoid of adjuvants (L(+)-O-PS14,23F) was also found to produce antibodies at a titer which was higher than that in the control (FCA/FIA) and similar to that in the adjuvant-containing liposomes. The recruitment of an adjuvant provided better results, but the liposome devoid of adjuvants was also found to be useful as a vaccine.

### EXPERIMENTAL EXAMPLE 5: Challenge Test

For comparison with the immunopotentiating liposome of the present invention, the commercially available pneumonia vaccine Prevenar-7 (Wyeth) was used in a challenge test on mice.

BALB/C female mice 12 weeks old were divided into groups of six. They were immunized with a sham (negative control), Prevenar-7 (positive control), L(+)-MPL-PS14 prepared in Example 2, and L(+)-MPL-PS14,23F prepared in Example 5. As for the antigen dose, L(+)-MPL-PS14 was used in an amount of PS 5 µg/monovalent (serotype 14), L(+)-PS14,23F MPL in an amount of PS 5 µg/serotype 14 and PS 5 µg/serotype 23F, and Prevenar in an amount of 0.4 µg/serotype 14, in a total amount of 3.2 µg/heptavalent.

Three weeks after tertiary immunization, a challenge test was conducted.

*Streptococcus pneumoniae* serotype 14 (*Streptococcus pneumoniae* (Klein) Chester ATCC 6314) was cultured and intraperitoneally injected once at a dose of 0.64 x 10⁸ CFU into the mice. Thereafter, their viability was monitored every day.

The results are shown in FIG. 5.

As shown in FIG. 5, only one of the six mice in the negative control group survived after the injection. The groups immunized with the immunopotentiating liposomes (administered with L(+)-MPL-PS14 and L(+)-MPL-PS14,23F), although different in survival rate depending on antigen dose, promised far higher viability than did Prevenar-7. Accordingly, the liposome vaccines according to the present invention are superior in vaccination activity to the commercially available heptavalent vaccine.

## Claims

1. A vaccine composition for use in prophylaxis of pneumonia, comprising pneumococcal capsular polysaccharide antigen-encapsulated cationic liposomes, said cationic liposomes containing no carrier proteins and being made of phosphatidylcholine, cholesterol, stearylamine and monophosphoryl lipid A (MPL) at a weight ratio of 5 ~ 7 : 2 ~ 4 : 0.5 ~ 2 : 0.02 ~ 0.2.

2. The vaccine composition for use of claim 1, wherein the cationic liposomes are made of phosphatidylcholine, cholesterol, stearylamine and monophosphoryl lipid A (MPL) at a weight ratio of 6 : 3 : 1 : 0.03.

3. The vaccine composition for use of claim 1, wherein the pneumococcal capsular polysaccharide antigen is used at a weight ratio of 1: 50 ~ 250 to phosphatidylcholine.

4. The vaccine composition for use of claim 1, wherein the cationic liposomes range in mean diameter from 50 to 2,000 nm.

5. The vaccine composition for use of claim 1, being formulated into a dosage form administered via a route selected from the group consisting of subcutaneous, intravenous, intraperitoneal, intramuscular, intranasal, inhalation, intravaginal, and oral routes.

6. The vaccine composition for use of claim 1, further comprising a pharmaceutically acceptable diluent or excipient.

## Patentansprüche

1. Vakzinzusammensetzung zur Verwendung für die Prophylaxe von Pneumonie, umfassend kapselförmiges Pneumokokken-Polysaccharid-Antigen gekapselt in kationischen Liposomen, wobei die kationischen Liposome keine Trägerproteine beinhalten und aus Phosphatidylcholin, Cholesterol, Stearylamin und Monophosphoryl-Lipid A (MPL) bei einem Gewichtsverhältnis von 5 ~ 7 : 2 ~ 4 : 0.5 ~ 2 : 0.02 ~ 0.2 gebildet sind.

2. Vakzinzusammensetzung zur Verwendung nach Anspruch 1, wobei die kationischen Liposome aus Phosphatidylcholin, Cholesterol, Stearylamin und Monophosphoryl-Lipid A (MPL) bei einem Gewichtsverhältnis von 6 : 3 : 1 : 0.03 gebildet sind.

3. Vakzinzusammensetzung zur Verwendung nach Anspruch 1, wobei das kapselförmige Pneumokokken-Polysaccharid-Antigen bei einem Gewichtsverhältnis von 1: 50 ~ 250 zu Phosphatidylcholin verwendet wird.

4. Vakzinzusammensetzung zur Verwendung nach Anspruch 1, wobei der mittlere Durchmesser der kationischen Liposome im Bereich von 50 bis 2,000 nm ist.

5. Vakzinzusammensetzung zur Verwendung nach Anspruch 1, formuliert als eine Dosierungsform, die über einen Weg ausgewählt aus der Gruppe bestehend aus subkutanen, intravenösen, intraperitonealen, intramuskulären, intranasalen, Inhalations-, intravaginalen, und oralen Wegen verabreicht wird.

6. Vakzinzusammensetzung zur Verwendung nach Anspruch 1, ferner umfassend ein pharmazeutisch akzeptables Verdünnungsmittel oder einen pharmazeutisch akzeptablen Hilfsstoff.

## Revendications

1. Composition vaccinale pour l'utilisation dans la prophylaxie de la pneumonie, comprenant des liposomes cationiques à antigène polysaccharidique capsulaire pneumococcique encapsulé, lesdits liposomes cationiques ne contenant pas de protéines vectrices et étant faits de phosphatidylcholine, de cholestérol, de stéarylamine et de monophosphoryl lipide A (MPL) dans un rapport pondéral de 5 ~ 7 : 2 ~ 4 : 0,5 ~ 2 : 0,02 ~ 0,2.

2. Composition vaccinale pour l'utilisation selon la revendication 1, dans laquelle les liposomes cationiques sont faits de phosphatidylcholine, de cholestérol, de stéarylamine et de monophosphoryl lipide A (MPL) dans un rapport pondéral de 6 : 3 : 1 0,03.

3. Composition vaccinale pour l'utilisation selon la revendication 1, dans laquelle l'antigène polysaccharidique capsulaire pneumococcique est utilisé dans un rapport pondéral de 1 : 50 - 250 à la phosphatidylcholine.

4. Composition vaccinale pour l'utilisation selon la revendication 1, dans laquelle les liposomes cationiques vont en diamètre moyen de 50 à 2 000 nm.

5. Composition vaccinale pour l'utilisation selon la revendication 1, qui est formulée sous une forme posologique administrée par une voie choisie dans le groupe constitué par les voies sous-cutanée, intraveineuse, intrapéritonéale, intramusculaire, intranasale, l'inhalation, intravaginale et orale.

6. Composition vaccinale pour l'utilisation selon la revendication 1, comprenant en outre un diluant ou excipient pharmaceutiquement acceptable.
